Europäisches Patentamt

European Patent Office

Office européen des brevets

(19)

(11) EP 0 745 116 B1

(12) **EUROPÄISCHE PATENTSCHRIFT**

(45) Veröffentlichungstag und Bekanntmachung des Hinweises auf die Patenterteilung:
**18.11.1998 Patentblatt 1998/47**

(21) Anmeldenummer: **95909690.0**

(22) Anmeldetag: **09.02.1995**

(51) Int Cl.6: **C11C 3/12**

(86) Internationale Anmeldenummer:
**PCT/EP95/00456**

(87) Internationale Veröffentlichungsnummer:
**WO 95/22591 (24.08.1995 Gazette 1995/36)**

(54) **HÄRTEN VON UNGESÄTTIGTEN FETTEN, FETTSÄUREN ODER FETTSÄUREESTERN**

HYDROGENATION OF UNSATURATED FATS, FATTY ACIDS OR FATTY ACID ESTERS

HYDROGENATION DE GRAISSES, D'ACIDES GRAS OU D'ESTERS D'ACIDES GRAS INSATURES

(84) Benannte Vertragsstaaten:
**BE CH DE DK ES FR GB IT LI NL PT SE**

(30) Priorität: **17.02.1994 DE 4405029**

(43) Veröffentlichungstag der Anmeldung:
**04.12.1996 Patentblatt 1996/49**

(73) Patentinhaber: **Degussa Aktiengesellschaft 60311 Frankfurt (DE)**

(72) Erfinder:
• **TACKE, Thomas**
  **D-61381 Friedrichsdorf (DE)**
• **WIELAND, Stefan**
  **D-63069 Offenbach (DE)**
• **PANSTER, Peter**
  **D-63517 Rodenbach (DE)**
• **BANKMANN, Martin**
  **D-63571 Gelnhausen (DE)**
• **BRAND, Reinhold**
  **D-63571 Gelnhausen (DE)**
• **MÄGERLEIN, Hendrik**
  **D-61476 Kronberg (DE)**

(56) Entgegenhaltungen:
**WO-A-94/06738          GB-A- 1 481 958**

• **DATABASE FSTA INTERNATIONAL FOOD INFORMATION SERVICE (IFIS), FRANFURT/MAIN, DE 73-07-N0360, N.G. KRUPENYA ET AL. 'Selectivity of the hydrogenation of cottonseed oil in saturated hydrocarbons, on a stationary catalyzer' & IZVESTIYA VYSSHIKH UCHEBNYKH ZAVEDENII, PISHCHEVAYA TEKHNOLOGIYA, Nr. 3, 1972 Seiten 67-69,**

## Beschreibung

Die vorliegende Erfindung betrifft ein Verfahren zum kontinuierlichen Härten von ungesättigten Fetten, Fettsäuren oder Fettsäureestern an einem geformten Katalysator im Festbett.

Nachwachsende Fette oder Öle tierischer oder pflanzlicher Herkunft gehören zu den Triglyceriden. Sie bilden einen wesentlichen Bestandteil der menschlichen Ernährung. Freie Fettsäuren können durch Spaltung der Triglyceride in Fettsäuren und Glycerin gewonnen werden. Die Fettsäuren aus pflanzlichen oder tierischen Quellen haben Kettenlängen von 12 bis 30 Kohlenstoffatomen. Es handelt sich zumeist um ungesättigte Fettsäuren mit bis zu drei Doppelbindungen. Die Doppelbindungen, insbesondere in dreifach ungesättigten Fettsäuren, sind der Grund für die geringe thermische Stabilität und leichte Oxidierbarkeit der ungesättigten Fettsäuren.

Mehrfach ungesättigte Fette sind für die menschliche Ernährung besonders wichtig, jedoch ist es häufig zur Verbesserung der Lagerstabilität und zur Verbesserung der Handhabbarkeit dieser Fette notwendig, die Doppelbindungen der mehrfach ungesättigten Fettsäuren selektiv bis auf eine Doppelbindung zu hydrieren. Man spricht dann von selektiver Härtung. Natürliche Fette liegen fast vollständig in der cis-isomeren Form vor. trans-isomere Fette sind physiologisch minderwertig. Sie stehen in Verdacht, zusammen mit den durchgehärteten Fetten den Triglycerid- bzw. Cholesterinspiegel des menschlichen Blutes zu erhöhen. Ziel der selektiven Härtung von Fetten ist es deshalb, die Bildung von trans-Isomeren sowie die Bildung vollständig durchgehärteter Fette zu unterdrücken.

Für Anwendungen in der Industrie müssen die Doppelbindungen möglichst vollständig durch Hydrierung bei gleichzeitiger Aufrechterhaltung des Säurecharakters der Fettsäuren entfernt werden. Diese vollständige Hydrierung der Doppelbindungen von Fettsäuren wird auch als Durchhärtung der Fettsäuren bezeichnet.

Der Sättigungsgrad von Fetten und Fettsäuren, d. h. ihr Gehalt an noch verbliebenen Doppelbindungen, kann durch die Jodzahl Tgl-64 (Wijs-Methode) des A.O.C.S. bestimmt werden. Natürliche Fette weisen je nach dem Grad der Sättigung Jodzahlen zwischen 150 (Sojaöl) und 50 (Rindertalg) auf.

Fette und Fettsäuren werden vorwiegend noch chargenweise bei Temperaturen von 100 - 300° C unter einem erhöhten Wasserstoffdruck von 1 - 35 bar in Anwesenheit eines geeigneten Katalysators hydriert. Hauptsächlich kommen hierfür Nickelkatalysatoren in Form von feinverteiltem Nickel entweder direkt oder auf Siliziumoxid als Träger in Frage. Neben diesen Nickelkatalysatoren sind jedoch auch Edelmetall-Trägerkatalysatoren für die selektive bzw. vollständige Härtung von Fetten, Fettsäuren und Fettsäureestern bekannt. Edelmetall-Trägerkatalysatoren sind insbesondere für die kontinuierliche Härtung von Fetten und Fettsäuren in Rieselbettreaktoren geeignet.

So beschreibt z. B. die DE 41 09 502 die kontinuierliche Härtung von Rohfettsäuren im Rieselbett an einem Palladium/Titanoxid-Katalysator. Die Reaktionsmedien werden dabei in Form eines 2-Phasen-Gemisches aus flüssigen Fettsäuren und Wasserstoff-Gas am Festbettkatalysator zur Reaktion gebracht. Die Hydrieraktivität in diesem Verfahren läßt dabei nur Raumgeschwindigkeiten von 1,2 $h^{-1}$ zu und sollte im Interesse einer höheren Wirtschaftlichkeit verbessert werden. Zudem hat sich gezeigt, daß die selektive Härtung im Rieselbett zur Bildung von trans-Isomeren neigt.

Aufgabe der vorliegenden Erfindung ist es deshalb, ein Verfahren zur kontinuierlichen Härtung von Fetten, Fettsäuren oder Fettsäureestern anzugeben, welches eine wesentlich verbesserte Hydrieraktivität aufweist. Das Verfahren soll sowohl für die selektive Härtung von eßbaren Fetten und Ölen mit geringer trans-Isomerenbildung geeignet sein, als auch für die vollständige Härtung von Fetten und freien Fettsäuren für technische Anwendungen.

Die Aufgabe wird durch ein Verfahren zum kontinuierlichen Härten von ungesättigten Fetten, Fettsäuren oder Fettsäureestern an einem geformten Katalysator im Festbett gelöst, welches dadurch gekennzeichnet ist, daß die Fette, Fettsäuren oder Fettsäureester zusammen mit dem für die Härtung benötigten Wasserstoff und in Gegenwart eines überkritischen Mediums oder Lösungsmittels über einen Katalysator geleitet und dabei umgesetzt werden und daß anschließend die Fette, Fettsäuren oder Fettsäureester durch Entspannen vom überkritischen Medium bzw. Lösungsmittel abgetrennt werden und damit als Reinsubstanz ohne Lösungsmittel vorliegen.

Überkritische Lösungsmittel bzw. Medien werden in vielen Bereichen der technischen Chemie als auch der Nahrungsmittelchemie eingesetzt. Haupteinsatzgebiet überkritischer Medien in der Nahrungsmittelchemie ist die Extraktion bestimmter Nahrungsmittelkomponenten aus natürlichen Rohstoffquellen. Bevorzugt wird dafür überkritisches Kohlendioxid eingesetzt, welches sich durch hohe Reinheit, gute Umweltverträglichkeit und relativ geringe Kosten auszeichnet. Das Härten von Fetten, Fettsäuren oder Fettsäureestern in überkritischen Medien ist bisher noch nicht bekannt geworden.

Das erfindungsgemäße Verfahren führt zu überraschend hohen Hydrieraktivitäten der eingesetzten Katalysatoren. Es hat sich gezeigt, daß die Hydrieraktivitäten gleichartiger Katalysatoren bei Anwendung im erfindungsgemäßen Verfahren um den Faktor 10 bis 50 größer sein können als beim Einsatz in der konventionellen Rieselbetthärtung. Darüber hinaus weist das erfindungsgemäße Verfahren eine geringere cis/trans-Isomerisierung auf.

Das Verfahren arbeitet besonders vorteilhaft bei Reaktionstemperaturen zwischen der kritischen Temperatur $T_{Kr.}$ des Lösungsmittels und dem zehnfachen Wert, bevorzugt zwischen $T_{Kr.}$ und $7 \cdot T_{Kr.}$, und bei Drucken zwischen dem 0,8-fachen des Druckes $P_{Kr.}$ des Lösungsmittels am kritischen Punkt und $6 \cdot P_{Kr.}$, bevorzugt zwischen $P_{Kr.}$ und $4 \cdot P_{Kr.}$.

Geeignete Lösungsmittel für das erfindungsgemäße Verfahren sind Aceton, Ammoniak, Butan, Kohlendioxid, Chloroform, Chlortrifluormethan, Dichlordifluormethan, Dichlorfluormethan, 1,2-Dichlortetrafluorethan, Ethan, Ethylmethylether, Methan, Stickstoffmonoxid, Distickstoffmonoxid, n-Pentan, Propan, Schwefelhexafluorid, Trichlorfluormethan und Xenon. Durch binäre oder ternäre Gemische dieser Lösungsmittel können die Lösungseigenschaften an den zu lösenden Stoff angepaßt werden. Eine weitere Steigerung des Lösungsvermögens und der Selektivität der Lösungseigenschaften überkritischer Lösungsmittel kann durch Zugabe geringer Mengen (bis ca. 2 Vol.%) sogenannter Modifikatoren erreicht werden. Als Modifikatoren eignen sich Alkohole (Methanol, Ethanol), Aldehyde, Ketone, Säuren, Kohlenwasserstoffe sowie Fluor/Chlor-Kohlenwasserstoff und Wasser.

Besonders geeignet sind Lösungsmittel bzw. Lösungsmittelgemische, deren kritische Temperatur im Bereich zwischen -120° C und 250° C liegt bei kritischen Drucken zwischen 20 und 200 bar und die eine Dichte am kritischen Punkt größer als 0,1 $g/cm^3$ aufweisen.

Bevorzugt eignen sich Kohlendioxid, Stickstoffmonoxid, Distickstoffmonoxid, Propan und Pentan mit Dichten am kritischen Punkt zwischen 0,2 und 0,5 $g/cm^3$. Sie weisen ein gutes Lösevermögen für organische Materialien auf. Unter den Reaktionsbedingungen des Verfahrens nehmen die Dichten des überkritischen Lösungsmittels mit steigendem Druck im Reaktor deutlich zu. Dadurch verbessert sich ihr Lösungsvermögen weiter. Im Falle von Kohlendioxid verdoppelt sich zum Beispiel die Dichte von etwa 0,5 $g/cm^3$ auf etwa 1 $g/cm^3$ bei Erhöhung des Druckes von $P_{Kr}$ auf 5 · $P_{Kr}$ (jeweils bei der kritischen Temperatur).

Die kritischen Temperaturen liegen zwischen -94° C für Stickstoffmonoxid und 196,5° C für Pentan und ermöglichen damit eine besonders schonende Behandlung von organischen Materialien. Bevorzugt werden Kohlendioxid mit einer kritischen Temperatur von 31° C, einem kritischen Druck von 72,8 bar und einer kritischen Dichte von 0,467 $g/cm^3$ sowie Distickstoffmonoxid mit einer kritischen Temperatur von 36,4° C, einem kritischen Druck von 71,5 bar und einer kritischen Dichte von 0,452 $g/cm^3$ eingesetzt. Die Löseeigenschaften von Kohlendioxid können durch Mischen mit Propan vergrößert werden (z.B. Mischung aus 75 Volumenanteilen Kohlendioxid und 25 Volumenanteilen Propan).

Für das erfindungsgemäße Verfahren können alle bekannten Hydrierkatalysatoren eingesetzt werden, also auch z. B. Nickel-, Platin-, Palladium-, Rhodium-, Ruthenium-Katalysatoren oder Kombinationen hiervon auf $SiO_2$, $Al_2O_3$, $TiO_2$, $ZrO_2$, MgO, Aktivkohle oder auf Mischungen hiervon wie z. B. MgO x $Al_2O_3$. Besonders bewährt haben sich die Platingruppenmetalle auf geformten Trägern. Die katalytische Aktivität kann durch Promotoren beeinflußt werden. So ist z. B. bekannt, daß Silber als Promotor für Nickel- und Palladium-Katalysatoren die Bildung von trans-Isomeren vermindert. In der Technik werden insbesondere sulfidierte Nickelkatalysatoren eingesetzt.

Die Träger sollten eine hohe spezifische Oberfläche aufweisen, um eine gute Dispersion der Katalysatormetalle zu ermöglichen. Vorteilhaft sind spezifische Oberflächen zwischen 10 und 1000 $m^2/g$. Besonders wichtig für das erfindungsgemäße Verfahren ist auch die Porenstruktur der Träger. Sie sollten ein Gesamtporenvolumen zwischen 0,05 und 6,5 ml/g aufweisen, welches sich überwiegend aus Meso- und Makroporen zusammensetzt. Mikroporen sind unerwünscht und sollten nur einen geringen Prozentsatz am Gesamtporenvolumen ausmachen.

Die Begriffe Mikro-, Meso- und Makroporen werden hier in Übereinstimmung mit den Definitionen der IUPAC verwendet. Gemäß diesen Definitionen umfassen die Porengruppen folgende Durchmesserbereiche:

| | |
|---|---|
| Mikroporen | $d < 2$ nm |
| Mesoporen | $d = 2 \dots 50$ nm |
| Makroporen | $d > 50$ nm |

Meso- und Makroporen garantieren durch ihre großen Porendurchmesser eine optimale Zugänglichkeit der auf ihren Oberflächen abgeschiedenen katalytisch aktiven Edelmetallkristalle für die Fett-, Fettsäure- bzw. Fettsäureester-Moleküle. Unterstüzt wird diese Zugänglichkeit durch die Tatsache, daß die verwendeten überkritischen Lösungsmittel eine geringe Viskosität aufweisen.

Der Gehalt an Platingruppen-Metallen auf dem Träger sollte zwischen 0,05 und 5 Gew.-% betragen, bevorzugt zwischen 0,1 und 3,0 Gew.-%.

Die Platingruppen-Metalle müssen auf dem Träger fein verteilt abgeschieden werden, um eine möglichst große Metalloberfläche für den katalytischen Prozeß zur Verfügung zu stellen. Ein Maß für die Größe der katalytisch aktiven Metalloberfläche ist die Adsorption von Kohlenmonoxid. Sie sollte in Abhängigkeit vom Gehalt an Platingruppen-Metallen zwischen 0,05 und 5,0 ml CO/g der fertigen Katalysatorkörper liegen. Wird vorausgesetzt, daß ein Edelmetallatom ein CO-Molekül adsorbiert und letzteres sich wie ein ideales Gas verhält mit einer angenommenen Projektionsfläche von 6,25 x $10^{-20}$ $m^2$/Molekül, so läßt sich aus den o.a. Werten eine aktive Oberfläche der Platingruppen-Metalle auf dem fertigen Katalysator von ca. 0,1 - 10 $m^2/g$ Katalysator errechnen.

Die Katalysatorträger können beliebig geformt sein. Geeignet sind insbesondere alle für Festbett-Katalysatoren bekannten Formen, also Kugeln, Zylinder, Hohlzylinder und Speichenräder sowie monolithische Katalysatorträger in Form von Wabenkörpern mit parallelen Strömungskanälen oder Schaumkeramiken mit einem offenen Porensystem.

Die monolithischen Wabenkörper können durchgängig aus dem hochoberflächigen Trägermaterial bestehen (Vollkatalysator) oder aus einem inerten Tragkörper mit einer Beschichtung aus dem hochoberflächigen Trägermaterial aufgebaut sein (Beschichtungskatalysator).

Ein besonderer Vorteil des erfindungsgemäßen Verfahrens ist es, daß verglichen mit konventionellen Verfahren kleinteilige Katalysatorträger als Schüttgut eingesetzt werden können, ohne daß der Druckabfall über das Katalysatorbett zu groß wird. Dies wird durch die geringe Viskosität des überkritischen Lösungsmittels ermöglicht. Vorteilhaft können deshalb Katalysatorträger eingesetzt werden mit äußeren Abmessungen im Bereich zwischen 0,1 und 3,0 mm, insbesondere zwischen 0,2 und 1,0 mm. Dadurch lassen sich sehr hohe Katalysatoraktivitäten erzielen. Bevorzugt werden kugelförmige Träger verwendet.

Wegen der geringen Abmessungen der Katalysatoren weisen sie in der Schüttung eine sehr hohe geometrische Oberfläche relativ zum Gesamtvolumen der Schüttung auf. Dies kommt der katalytischen Aktivität der Katalysatorschüttung zugute.

Weiter verbessert werden kann diese Aktivität, wenn die Platingruppenmetalle auf diesen Trägern in einer äußeren Schale von 10 - 40 µm aufgebracht sind. Die Schalenimprägnierung ist besonders für die selektive Fetthärtung von Bedeutung. Sie verhindert nämlich, daß Fettmoleküle, die in das Innere des Katalysatorträgers hineindiffundiert sind, dort lange mit katalytisch aktiven Metallen in Berührung stehen und somit voll durchgehärtet werden. Für die vollständige Härtung von Fetten bzw. Fettsäuren können dagegen auch voll durchimprägnierte Katalysatorträger eingesetzt werden.

Als Katalysatorträger eignen sich verschiedene Materialien. Sie müssen allerdings die obengenannten Forderungen an ihre physikalischen Eigenschaften erfüllen und gegenüber den Reaktionsmedien, insbesondere gegenüber den Fettsäuren, beständig sein. Bei der konventionellen Fetthärtung haben sich Aktivkohle, Siliziumdioxid, Aluminiumoxid, Aluminium/Silizium-Mischoxide, Bariumsulfat, Titanoxid, mit Titanoxid beschichtete Glasperlen und Ionenaustauscherharze bewährt. Diese Trägermaterialien können auch im erfindungsgemäßen Verfahren eingesetzt werden. In optimaler Weise werden die genannten Forderungen aber von Organosiloxanamin-Copolykondensaten oder von polymeren, sekundären und/oder tertiären OrganosiloxanaminVerbindungen oder von Organosiloxan-Polykondensaten erfüllt. Diese Trägermaterialien werden in den deutschen Patentschriften DE 38 00 563 C1, DE 38 00 564 C1, DE 39 25 359 C1 und DE 39 25 360 C1 bzw. der noch nicht veröffentlichten Patentanmeldung P 42 25 978.1 beschrieben. Platingruppenmetall-haltige Katalysatoren auf diesen Trägern werden in den Patentschriften DE 41 10 705 C1 und DE 41 10 706 C1 offenbart.

Die folgenden Beispiele verdeutlichen die Wirkungsweise des erfindungsgemäßen Verfahrens zum Hydrieren von Fetten, Fettsäuren oder Fettsäureestern.

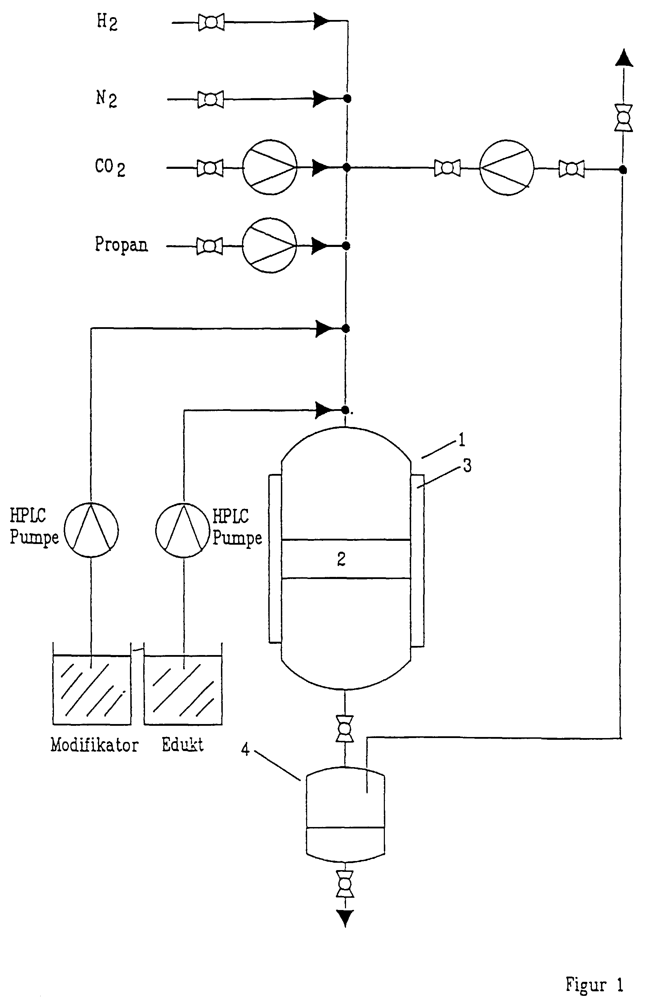

Figur 1 zeigt die schematische Darstellung der für die Beispiele benutzten Hydrierapparatur. (1) bezeichnet den Hydrierreaktor. Es handelt sich um ein 840 mm langes Edelstahlrohr mit einem Innendurchmesser von 15,7 mm. Dieses Edelstahlrohr ist etwa auf halber Höhe mit einer Katalysatorschicht (2) von 10 - 30 mm Höhe befüllt. Ober- und unterhalb der Katalysatorschüttung befinden sich Stopfen aus Quarzwolle. Sie trennen das eigentliche Katalysatorbett von Glasperlen, die das freie verbleibende Volumen des Edelstahlrohres ober- und unterhalb der Katalysatorschüttung auffüllen. Die Inertschüttung oberhalb der Katalysatorschüttung dient gleichzeitig zum Vermischen des überkritischen Mediums bzw. Lösungsmittels mit den Reaktanden.

Der Reaktor besitzt einen äußeren Mantel (3), der zur Einstellung der Reaktionstemperatur im Gegenstrom von Siliconöl als Heizmedium durchflossen wird. Diese Anordnung garantiert, daß der Temperaturgradient über das Katalysatorbett sehr gering ist.

Die für das Verfahren benötigten Medien werden dem Reaktor von oben zugeführt. Stickstoff wird lediglich zum Spülen des Reaktors vor und nach einer Hydrierung eingesetzt. Dem überkritischen Gemisch aus Wasserstoff, Kohlendioxid und gegebenenfalls Propan wird oberhalb des Reaktors noch ein eventuell benötigter Modifikator und das zu hydrierende Edukt zudosiert.

Im Gegensatz zum konventionellen Rieselbettverfahren ist die hier gewählte senkrechte Aufstellung des Reaktorrohres nicht zwingend.In einer möglichen Ausführungsform des Verfahrens bei hinreichender Verdünnung der Reaktanden im überkritischen Medium bzw. Lösungsmittel existiert eine nahezu homogene Phase, die bei jeder beliebigen Orientierung des Reaktorrohres über das Katalysatorbett gepumpt werden kann. Die senkrechte Aufstellung wurde hier nur zur Vereinfachung der Beschreibung gewählt.

Nach Durchlaufen des Reaktors gelangen die Reaktionsmedien in einen Separator (4). In diesem Separator wird das Reaktionsgemisch aus Produkt, gegebenenfalls überschüssigem Wasserstoff und überkritischem Lösungsmittel durch Entspannen auf Drucke unterhalb des kritischen Druckes in ein Zweiphasengemisch überführt. Beim Entspannen gehen das Lösungsmittel und der Wasserstoff in den gasförmigen Zustand über, wodurch das Lösungsvermögen des Lösungsmittels praktisch auf Null verringert wird. Das Produkt der Hydrierreaktion scheidet sich deshalb als Flüssigkeit oder Feststoff aus dem Reaktionsgemisch aus und kann damit von dem gasförmigen Lösungsmittel und dem restlichen Wasserstoff getrennt werden. Das jetzt gasförmige Lösungsmittel und der restliche Wasserstoff können entweder an die Atmosphäre abgegeben werden oder wieder komprimiert und in den Prozeß rezykliert werden. Das Entspannen des Reaktionsmediums nach Durchlaufen des Reaktors kann auch in mehreren Stufen unter Druckabnahme realisiert werden. Damit können die Reaktionsprodukte in mehreren Fraktionen, je nach Löslichkeit im überkritischen Medium, abgeschieden werden.

Eine gegebenenfalls notwendige Stofftrennung z. B. durch Destillation kann damit eingespart werden.

Die Hydrierapparatur von Figur 1 wurde in den folgenden Beispielen für die kontinuierliche Hydrierung von Ethylestern verschiedener Fettsäuren eingesetzt, deren Hauptbestandteil der Linolsäure-Ethylester war. Das Edukt hatte im einzelnen folgende Zusammensetzung:

Tabelle 1:

| Zusammensetzung des Eduktes | |
| --- | --- |
| Linolsäure-Ethylester C18:2 | 76,8 Gew.-% |
| Ölsäure -Ethylester | |
| cis-Form C18:1(c) | 13,2 Gew.-% |
| trans-Form C18:1(t) | 0 Gew.-% |
| Stearinsäure-Ethylester C18:0 | 2,7 Gew.-% |
| Palmitinsäure-Ethylester C16:0 | 7,3 Gew.-% |

Als überkritisches Lösungsmittel wurde reines Kohlendioxid bzw. ein Kohlendioxid-Propan-Gasgemisch verwendet.

Linolsäure-Ethylester ist ein Ester der zweifach ungesättigten Linolsäure mit 18 Kohlenstoffatomen.

Die Doppelbindungen dieser Fettsäure werden in einer Folgereaktion, also nacheinander hydriert. Im Reaktionsprodukt finden sich deshalb neben Resten der Linolsäure C18:2 die einfach ungesättigte Ölsäure C18:1 und die vollständig gesättigte Stearinsäure C18:0. Die einfach ungesättigte Ölsäure kann in zwei isomeren Formen vorkommen, nämlich als cis-Form C18:1(c) und als trans-Form C18:1(t). Ölsäure aus natürlichen Quellen weist überwiegend die cis-Form auf. Während des Hydrierens wird die Ölsäure teilweise zur trans-Form isomerisiert.

Zur Analyse des Reaktionsproduktes wurde die Flüssigkeit stündlich aus dem Separator entfernt und eine Probe davon in einem Gaschromatographen untersucht und die gebildeten Reaktionsprodukte identifiziert und quantitativ bestimmt. Aus diesen Messungen konnte die Selektivität der Bildung von Ölsäure gegenüber Stearinsäure ermittelt werden sowie der Grad der cis/trans-Isomerisierung.

Als Maß für die integrale Aktivität A der Katalysatoren in dem erfindungsgemäßen Verfahren wurde a) die Jodzahl-Abnahme, normiert auf eine Stunde, b) die spezifische Jodzahl-Abnahme, normiert auf eine Stunde und 1 g aktives Metall, sowie c) die spezifische Hydrieraktivität in Anzahl der Mole Wasserstoff, die pro Gramm aktives Metall aM und pro Stunde umgesetzt wurden, aus der Jodzahl der Proben berechnet. Die Jodzahl (JZ) ist ein Maß für die Anzahl der im Produkt noch nicht abgesättigten Doppelbindungen und wird in Gramm Jod, die von 100 g der Proben absorbiert werden, angegeben. Sie wird gemäß der offiziellen Methode Tg1-64 (Wijs-Methode) des A.O.C.S. bestimmt. Aus der Jodzahl $JZ_{Edukt}$ des Eduktes und der Jodzahl $JZ_{Produkt}$ des Produktes berechnet sich die spezifische Hydrieraktivität A zu

$$A = \frac{(JZ_{Edukt} - JZ_{Produkt}) \times \Phi}{100 \times g\ aM \times M_{Jod}} \quad \left[\frac{mol\ H_2}{g\ aM \times h}\right]$$

$\Phi =$      Massendurchfluß des Edukts in [g/h]

$g\ aM =$      Gramm aktives Metall [g]

$M_{Jod} =$      Molmasse von Jod in [g Mol]

Die spezifische cis/trans-Isomerisierung B wird dimensionslos angegeben als Prozent trans-Isomer im gebildeten Produkt nach GC-Analytik in Relation zur Jodzahl-Abnahme.

$$B = \frac{\%\text{ - trans - Isomer}}{(JZ_{Edukt} - JZ_{Produkt})}$$

EP 0 745 116 B1

Es kamen vier verschiedene Katalysatorsysteme zum Einsatz, die mit ihren Eigenschaften in Tabelle 2 aufgeführt sind. Beim Pd/C-Katalysator handelt es sich um einen Schalenkatalysator (20 μm Schale) auf einer mesoporösen Kugelkohle. Pd/OFP bezeichnet einen Palladium-Katalysator auf einem Träger aus einem organofunktionellen Polysiloxan gemäß Beispiel 2 der Patentschrift DE 41 10 706 C1.

Als Unedelmetall-Katalysator kam der kommerzielle Katalysator RCH Ni 55/5 TST von Hoechst zum Einsatz. Dabei handelt es sich um einen Trägerkatalysator mit einem Gehalt von etwa 54 Gew.-% Nickel auf 4 Gew.-% Mangan enthaltendem Kieselguhr.

In Tabelle 2 sind die untersuchten Katalysatorsysteme durch Angaben zur Form und Größe des Trägermaterials sowie durch Angaben zu seiner Porenstruktur charakterisiert. Bezüglich des Nickel-Katalysators enthält die Tabelle nur die aus den Datenblättern entnehmbaren Parameter.

Die in Tabelle 2 angegebenen Porenvolumina wurden im Falle von Mikro- und Mesoporen durch Auswertung von Stickstoff-Adsorptionsisothermen nach DIN 66133 bestimmt. Das Porenvolumen der Makroporen wurde durch Hg-Porosimetrie ebenfalls nach DIN 66133 ermittelt.

Tabelle 2 enthält weiterhin Angaben über die Art der Verteilung der Platingruppen-Metalle über den Querschnitt der Katalysatorträger und zur Feinteiligkeit der Platingruppenmetalle gemessen durch ihre Kohlenmonoxidadsorption.

## Tabelle 2   Eigenschaften der Katalysatoren

| Kataly-sator | Träger | Form | Größe [mm] | Poren [ml/g] | | | |
|---|---|---|---|---|---|---|---|
| | | | | Mikro | Meso | Makro | total |
| Pd/C | C | Kugeln | 0,4-0,8 | 0,19 | 0,42 | 0,14 | 0,75 |
| Pd/OFP | OFP | Kugeln | 0,3-0,8 | – | 1,54 | 0,72 | 2,26 |
| Pt/OFP | OFP | Kugeln | 0,4-0,8 | – | 1,48 | 0,68 | 2,16 |
| Ni/SiO$_2$ | SiO$_2$ | Granulat | 0,45-1,0 | | | | 0,5 |

| Kataly-sator | katalytisches Metall | Metall Verteilung | Metall Gehalt [%] | CO-Aufnahme [ml CO/g Kat] |
|---|---|---|---|---|
| Pd/C | Pd | 20 μm Schale | 0,5 | 0,39 |
| Pd/OFP | Pd | 20 μm Schale | 1,0 | 0,65 |
| Pt/OFP | Pt | durchim-prägniert | 2,0 | 0,22 |
| Ni/SiO$_2$ | Ni | homogen | 54 | |

Beispiel 1

Das in Tabelle 1 charakterisierte Edukt aus Ethylestern verschiedener Fettsäuren wurde erfindungsgemäß in Gegenwart eines überkritischen Mediums unter Verwendung der Katalysatoren der Tabelle 2 bei den in Tabelle 3 angegebenen Reaktionsbedingungen hydriert. Die in Tabelle 3 angegebene Raumgeschwindigkeit (LHSV = liquid hourly space velocity) ist das stündlich pro Katalysatorvolumen dosierte Flüssigkeitsvolumen des Reaktionseduktes.

Die Ergebnisse bezüglich spezifischer Hydrieraktivität A, Jodzahl-Abnahme pro Stunde, spezifischer Jodzahl-Abnahme pro Stunde und der spezifischen cis/trans-Isomerisierung sind in Tabelle 3 aufgeführt. Tabelle 3 enthält außerdem einen Vergleich mit Rieselbetthärtungen aus verschiedenen Literaturquellen.

Wie Tabelle 3 zeigt, können in Gegenwart eines überkritischen Mediums bzw. Lösungsmittels in der Härtung von Fetten und Ölen, Fettsäuren bzw. Fettsäureestern mit geeigneten Katalysatoren viel bessere Aktivitäten und auch geringere cis/trans-Isomerisierungen als in der bekannten kontinuierlichen Rieselbetthärtung erzielt werden. Katalysator 2 (Pd/OFP) weist bei deutlich verminderter Bildung von trans-Isomeren im Vergleich zu kommerziellen Pd/C-(Kat. 5) bzw. Ni/SiO$_2$- (Kat. 6) Katalysatoren 65-fach bzw. 292-fach bessere metallspezifische Hydrieraktivitäten auf.

6

In bezug auf die metallspezifische Jodzahl-Abnahme sind die Faktoren 149 bzw. 837. Auch gegenüber dem Pd/Polystyrol-Katalysator (Kat. 4) ergeben sich im erfindungsgemäßen Verfahren noch um Größenordnungen höhere Aktivitäten, aber auch deutlich geringere cis/trans-Isomerisierungen. Die Pd/C- (Kat. 1) bzw. Pt/OFP- (Kat. 3) Katalysatoren weisen im erfindungsgemäßen Verfahren ebenfalls sehr gute Aktivitäten bzw. Selektivitäten auf, besser als vergleichbare Katalysatoren in der Rieselbetthärtung (Kat.4 - 9). Diese Ergebnisse wurden zudem noch bei 60° C erzielt, während die meisten der anderen zitierten Versuche bei deutlich höherer Temperatur durchgeführt wurden.

Aus der Literatur ist bekannt, daß Platin als Aktivkomponente bei der Hydrierung von Fetten, Fettsäuren und Fettsäureestern wenig geeignet ist. Aus Tabelle 3 ist jedoch ersichtlich, daß der Pt/OFP-Katalysator in Gegenwart eines überkritischen Mediums oder Lösungsmittel durchaus gute Hydrieraktivitäten aufweist und sich besonders durch eine geringe Bildung von trans-Isomeren auszeichnet.

Palladium-Katalysatoren sind dagegen in der Rieselbetthärtung für die Bildung von trans-Isomeren bekannt (siehe Katalysatoren 4 und 5 in Tabelle 3). Im erfindungsgemäßen Hydrierverfahren ist die Bildung von trans-Isomeren durch die Palladium-Katalysatoren jedoch stark vermindert.

Der kommerzielle Nickel-Katalysator (Katalysator 10) wurde sowohl im erfindungsgemäßen Hydrierverfahren als auch im konventionellen Rieselbettverfahren eingesetzt. Im konventionellen Rieselbettverfahren wurde bei 170°C, einem Wasserstoffdruck von 20 bar und einer Raumgeschwindigkeit von 5 h$^{-1}$ gearbeitet. Im erfindungsgemäßen Verfahren konnte die Temperatur auf 120°C gesenkt werden. Trotzdem wurde eine um 25 bis 30 % höhere Hydrieraktivität bei deutlich verminderter cis/trans-Isomerisierung beobachtet.

Die Angaben in Tabelle 3 weisen den Vorteil der erfindungsgemäßen Hydrierung in Gegenwart überkritischer Medien bzw. Lösungsmittel nach. Die Katalysatoren auf OFP-Trägern mit ihrer optimalen Porenstruktur führen dabei zu besonders guten Resultaten.

Während die Katalysatoren 1,2 und 3 für technische Anwendungen des erfindungsgemäßen Hydrierverfahrens trotz ihres kleinen Partikeldurchmessers gut geeignet sind, ist das für die Katalysatoren 4, 5 und 6 bei der konventionellen Rieselbetthärtung nicht der Fall. Ihre Partikeldurchmesser sind für dieses Verfahren zu klein und führen zu einem hohen Druckverlust im Rieselbett. Typische Korngrößen für die Anwendung im Rieselbett liegen deshalb bei 1 bis 5 mm und haben ein weiteres Absinken der spezifischen Hydrieraktivität gegenüber den Werten der Katalysatoren 4, 5 und 6 in Tabelle 3 zur Folge.

Das erfindungsgemäße Hydrierverfahren arbeitet dagegen mit einem Reaktionsgemisch aus überkritischem Medium bzw. Lösungsmittel, Wasserstoff und den zu hydrierenden Fetten, Fettsäuren oder Fettsäureestern, welches aufgrund der überkritischen Bedingungen für das Lösungsmittel eine geringe Viskosität besitzt und deshalb auch bei kleinen Partikeldurchmessern im Bereich zwischen 0,1 und 1 mm zu keinem übermäßigen Druckverlust im Katalysatorbett führt.

EP 0 745 116 B1

Tabelle 3   Vergleich verschiedener Katalysatorsysteme in der überkritischen bzw. Rieselbetthärtung

| Kat.-Nr. | Katalysator-bezeichnung/ Korndurch-messer | Hydrier-aktivität A | JZ-Abnahme [1/h] | spezifische JZ-Abnahme [1/hxg aM] | cis/trans-Isomerisie-rung B | Reaktionsparameter | Quelle |
|---|---|---|---|---|---|---|---|
| 1 | 0,5 % Pd/C 0,3 - 0,8 mm | 3,2 | 270 | 46154 | 0,23 | 60° C, 100 bar $CO_2$ + überstöchiom. $H_2$ LHSV 10 $h^{-1}$ Linolsäureethylester | gemäß Erfindung |
| 2 | 1 % Pd/OFP 0,4 - 0,8 mm | 14,3 | 1151 | 209273 | 0,11 | | |
| 3 | 2 % Pt/OFP 0,4 - 0,8 mm | 1,5 | 230 | 20909 | 0,08 | | |
| 10 | 54% Ni/$SiO_2$ 0,45-1,0 mm | 0,04 | 512 | 560 | 0,12 | 120°C, 100 bar $CO_2$, LHSV 5 $h^{-1}$ + überstöchiom. $H_2$ Linolsäureethylester | gemäß Erfindung |
| 10 | " | 0,03 | 429 | 470 | 0,25 | 170°C, 20 bar $H_2$, LHSV 5 $h^{-1}$ Linolsäureethylester | Rieselbett-härtung |
| 4 | 4 % Pd/Poly-styrol 0,3 - 0,8 mm | 0,53 | 270 | 3375 | 0,3 | 100°C, 3,45 bar $H_2$ LHSV 6 $h^{-1}$, Sojabohnenöl | JAOCS |
| 5 | 1 % Pd/C 0,18-0,42 mm | 0,22 | 28 | 1400 | 1,5 | 100°C, 3,45 bar $H_2$ LHSV 14 $h^{-1}$, Sojabohnenöl | Vol. 66 No. 7 |
| 6 | 50 % Ni/$SiO_2$ 0,03 mm | 0,049 | 250 | 250 | 0,4 | 150°C, 3,45 bar $H_2$, LHSV 10 $h^{-1}$, Sojabohnenöl | July 1989 |
| 7 | 2 % Pd/$TiO_2$ | 0,12 | 57,8 | 2890 | – | 170°C, 20 bar $H_2$, LHSV 1,07, dest. Fettsäure | DE 4109502 |
| 8 | 2 % Pd/C | 0,23 | 57,2 | 5720 | – | | Degussa AG |
| 9 | 0,5% Pd/$Al_2O_3$ | 0,10 | 48 | 9600 | – | 60°C, 21,1 bar $H_2$ LHSV 1 $h^{-1}$ Fettsäure (Ölsäure) | DOS 2310985 |

<u>Beispiel 2</u>

Mit dem Pd/OFP-Katalysator Nr. 2 von Tabelle 3 wurde ein direkter Vergleich zwischen der konventionellen Rieselbetthärtung und der erfindungsgemäßen Härtung in Gegenwart eines überkritischen Mediums bzw. Lösungsmittels durchgeführt.

Beide Versuche wurden unter exakt gleichen Reaktionsbedingungen in der beschrieben Hydrierapparatur vorgenommen. Zur Simulation der konventionellen Rieselbetthärtung wurde das überkritische Lösungsmittel $CO_2$ durch Stickstoff ersetzt. Die Raumgeschwindigkeit (LHSV) bei den Versuchen war jeweils 15 $h^{-1}$. Die Ergebnisse sind in Tabelle 4 aufgelistet.

Der Pd/OFP-Katalysator liefert auch in der konventionellen Rieselbetthärtung unter erhöhtem Stickstoffdruck sehr gute Aktivitäten und weist eine geringe Neigung zur Bildung von trans-Isomeren auf. Das liegt an den guten Diffusionseigenschaften des OFP-Trägers mit seiner nur aus Meso- und Makroporen bestehenden Porenstruktur.

Im erfindungsgemäßen Hydrierverfahren in Gegenwart eines überkritischen Mediums bzw. Lösungsmittels werden mit dem selben Katalysator jedoch noch wesentlich bessere Leistungsdaten erzielt.

Tabelle 4  Vergleich des erfindungsgemäßen Härtungsverfahrens unter überkritischen Bedingungen mit der konventionellen Rieselbetthärtung

| Kat.-Nr. | Katalysator-bezeichnung/ Korndurch-messer | Hydrier-aktivität A | JZ-Abnahme [1/h] | spezifische JZ-Abnahme [1/hxg aM] | cis/trans-Isomerisie-rung B | Reaktionsparameter | Verfahren |
|---|---|---|---|---|---|---|---|
| 2 | 1 % Pd/OFP | 22,8 | 1821 | 331091 | 0,078 | $60°$ C, 100 bar $CO_2$ + überstöchiom. $H_2$ LHSV 15 $h^{-1}$ Linolsäureethylester | nach Erfindung |
| 2 | 1 % Pd/OFP | 9,2 | 730,5 | 132818 | 0,226 | s.o. jedoch $N_2$ anstelle von $CO_2$ | konventio-nelles Rieselbett |

aM = aktives Metall

EP 0 745 116 B1

Beispiel 3

In einer dritten Versuchsreihe wurde die Abhängigkeit der Hydrieraktivität und der cis/trans-Isomerisierung von der Raumgeschwindigkeit bestimmt. Die Tabelle 5 enthält die Ergebnisse für die Raumgeschwindigkeiten (LHSV) 5, 10, 15, 30 und 60 $h^{-1}$.

Konventionelle Rieselbetthärtungen sind diffusions-limitiert, d. h. die Hydrierbarkeit wird durch die Diffusionsgeschwindigkeit der Reaktanden an die katalytisch aktiven Zentren und von ihnen weg begrenzt. Eine Erhöhung der Raumgeschwindigkeit führt deshalb zu keiner stärkeren katalytischen Umsetzung. Die Ergebnisse der Tabelle 5 zeigen dagegen, daß das erfindungsgemäße Hydrierverfahren selbst bei Raumgeschwindigkeiten von 60 $h^{-1}$ noch kinetisch kontrolliert ist, d. h. die katalytische Umsetzung wird nicht durch Diffusionsprozesse im Katalysator begrenzt, sondern hängt nur davon ab, mit welcher Geschwindigkeit das Reaktionsgemisch dem Katalysatorbett zugeführt wird.

Die Katalysatoraktivität nimmt daher mit steigender Raumgeschwindigkeit linear zu. Parallel dazu wird eine verminderte Bildung von trans-Isomeren beobachtet.

Oberhalb einer Raumgeschwindigkeit von 15 $h^{-1}$ nimmt die Katalysatoraktivität nicht mehr linear jedoch noch deutlich zu. Gleichzeitig werden geringfügig mehr trans-Isomere gebildet.

**Tabelle 5** Abhängigkeit der Aktivität und cis/trans-Isomerisierung von der Raumgeschwindigkeit bei der überkritischen Hydrierung mit Pd/OFP

| Kat.-Nr. | Katalysator-bezeichnung/ Korndurch-messer | LHSV [h$^{-1}$] | Hydrier-aktivität A | JZ-Abnahme [h$^{-1}$] | spezifische JZ-Abnahme [1/h·gaM] | cis/trans-Isomerisie-rung B | Reaktionsparameter |
|---|---|---|---|---|---|---|---|
| 2 | 1 % Pd/OFP 0,4 - 0,8 mm | 5 | 6,7 | 526 | 95636 | 0,161 | 60° C, 100 bar $CO_2$ + überstöchiom. $H_2$ Linolsäureethylester |
| 2 | 1 % Pd/OFP 0,4 - 0,8 mm | 10 | 14,3 | 1151 | 209273 | 0,105 | 60° C, 100 bar $CO_2$ + überstöchiom. $H_2$ Linolsäureethylester |
| 2 | 1 % Pd/OFP 0,4 - 0,8 mm | 15 | 22,8 | 1821 | 331091 | 0,078 | 60° C, 100 bar $CO_2$ + überstöchiom. $H_2$ Linolsäureethylester |
| 2 | " | 30 | 35,0 | 2581 | 566500 | 0,199 | " |
| 2 | " | 60 | 52,3 | 3862 | 847650 | 0,280 | " |

EP 0 745 116 B1

Beispiel 4

In einem Verfahrensvergleich zwischen dem erfindungsgemäßen Verfahren und der klassischen Rieselbetthärtung wurde Katalysator 2 zur selektiven Härtung von Sonnenblumenöl eingesetzt. Das eingesetzte Sonnenblumenöl wies folgende Zusammensetzung auf:

| | |
|---|---|
| $C_{18:3}$ | 1 Gew.-% |
| $C_{18:2}$ | 64, 8 Gew.-% |
| $C_{18:1}$ | 21,0 Gew.-% |
| Rest | gesättigte Fettsäuren mit unterschiedlicher Kettenlänge |

Als überkritisches Lösungsmittel wurde ein Gasgemisch aus 75 Vol.-% Kohlendioxid und 25 Vol.-% Propan eingesetzt. Die Ergebnisse dieser Versuchsreihe sind in Tabelle 6 dargestellt.

Auch in der selektiven Härtung von Triglyderiden (hier: Sonnenblumenöl) zeigt sich die Überlegenheit des erfindungsgemäßen Verfahrens sowohl hinsichtlich der Aktivität als auch hinsichtlich der Selektivität. Die Erhöhung der Hydrieraktivität mit der Raumgeschwindigkeit (LHSV) deutet darauf hin, daß die Reaktion nicht durch den Stofftransport limitiert ist. Es können ähnliche Hydrierkapazitäten wie in der selektiven Härtung von Linolsäureethylestern (s. Tabelle 5) erzielt werden.

Beispiel 5

In einem weiteren Verfahrensvergleich zwischen dem erfindungsgemäßen Verfahren und der klassischen Rieselbetthärtung wurden die Katalysatoren 2 und 10 zur Durchhärtung von Fettsäure eingesetzt. Die eingesetzte Fettsäure hatte die Jodzahl von 88,1 und eine Säurezahl von 202,0. Sie wies folgende Zusammensetzung auf:

| | |
|---|---|
| $C_{18:2}$ | 14,5 Gew.-% |
| $C_{18:1}$ | 77,5 Gew.-% |
| Rest | : gesättigte Fettsäuren mit unterschiedlicher Kettenlänge |

Die Säurezahl (SZ) dient zur Bestimmung des Gehalts an freien organischen Säuren in Fetten (Vorschrift s. Deutsches Arzneibuch 7. Auflg., 1968) und ist ein Maß für die Selektivität der Härtung. Die Säurezahl sollte während der Härtung möglichst konstant bleiben. Lediglich die Jodzahl (JZ) als Maßzahl für den Gehalt an ungesättigten Fettsäuren in Fetten sollte reduziert werden. Ziel der technischen Härtung ist die Verminderung der Jodzahl bis auf Werte unter 1 zur Verbesserung von Farbe, Geruch und Hitzestabilität

Mit dem Pd/OFP-Katalysator (siehe Tabelle 7) können in Gegenwart einer überkritischen Phase nahezu dreifach höhere Hydrieraktivitäten erzielt werden als in der Rieselbettphase. Auch die Säurezahl als Maßzahl für die Selektivität der Härtung bleibt in der überkritischen Härtung, offensichtlich durch die deutlich niedrigere Temperatur bedingt, auf einem höheren Niveau.

Die Hydrieraktivitäten des Pd/OFP-Katalysators in Gegenwart einer überkritischen Phase sind 34 - 79-fach höher als im Vergleich mit klassischen Katalysatoren (Nr. 7, 8 und 9) in der Rieselbettphase. Die Säurezahlen können in diesem Vergleich nicht berücksichtigt werden, da Fettsäuren unterschiedlicher Qualität mit unterschiedlichen Säurezahlen eingesetzt wurden.

Auch bei einer Raumgeschwindigkeit (LHSV) von 6,2 h$^{-1}$ lassen sich mit dem 1 % Pd/OFP-Katalysator noch Jodzahlen deutlich unter 1 erzielen.

Selbst mit klassischen Ni/$SiO_2$-Katalysatoren können in Gegenwart einer überkritischen Phase höhere Aktivitäten und Selektivitäten erzielt werden. Entscheidend hierfür ist wahrscheinlich die Reaktionsführung bei deutlich geringerer Temperatur, die eine verminderte Desaktivierung durch Nickel-Seifenbildung zur Folge hat.

Beispiel 6

Zur selektiven Härtung von Linolsäureethylester sowohl in der Rieselbettphase als auch in dem erfindungsgemäßen Verfahren wurde ein Cordierit Monolith mit einem Aluminiumoxid-Washcoat und einer Palladium-Belegung eingesetzt. Die Zellenzahl des Monolithen betrug 400 CPSI, entsprechend ca. 62 Zellen/cm$^2$. Der eingesetzte Monolith wies bei einem Katalysatorvolumen von 8,6 ml eine Pd-Belegung von 78 mg auf.

Die Versuchsergebnisse sind in Tabelle 8 dargestellt. Mit dem erfindungsgemäßen Verfahren läßt sich bei deutlich niedrigerer Temperatur sowohl eine höhere Aktivität als auch eine höhere Selektivität (niedrigere cis/trans-Isomerisierung) als in der Rieselbettphase erzielen.

Tabelle 6  Vergleich verschiedener Katalysatorsysteme in der überkritischen bzw. Rieselbetthärtung von Sonnenblumenöl

| Kat.-Nr. | Katalysator-bezeichnung/ Korndurch-messer | Hydrier-aktivität A | JZ-Abnahme [h$^{-1}$] | spezifische JZ-Abnahme [1/h·gaM] | cis/trans-Isomerisie-rung B | Reaktionsparameter | Verfahren |
|---|---|---|---|---|---|---|---|
| 2 | 1,0% Pd/OFP | 14,9 | 1087 | 209900 | 0,27 | 60°C, 100 bar CO$_2$/Propan + überstöchiom. H$_2$ LHSV 16,7 h$^{-1}$ | gemäß Erfindung |
| 2 | " | 21,4 | 1559 | 301100 | 0,21 | s.o., LHSV 26,3 h$^{-1}$ | gemäß Erfindung |
| 2 | " | 3,5 | 127,4 | 24600 | 0,32 | 60°C, 5 bar H$_2$ LHSV 14,9 h$^{-1}$ | Rieselbett |

EP 0 745 116 B1

EP 0 745 116 B1

Tabelle 7    Vergleich verschiedener Katalysatorsysteme in der überkritischen bzw. Rieselbetthärtung bei der Durchhärtung von Fettsäuren mit einer anfänglichen Jodzahl von 88,1 und einer Säurezahl von 202,0

| Kat.-Nr. | Katalysator-bezeichnung/ Korndurch-messer | Hydrier-aktivität A | JZ-Abnahme [1/h] | spezifische JZ-Abnahme [1/h · gaM] | End-Jodzahl | Säure-zahl | Reaktionsparameter | Verfahren |
|---|---|---|---|---|---|---|---|---|
| 2 | 1% Pd/OFP 0,4-0,8 mm | 7,9 | 458 | 112700 | 0,29 | 200,8 | 120°C, 140 bar $CO_2$, überstöchiom. $H_2$ LHSV 6,2 $h^{-1}$ | gemäß Erfindung |
| 2 | " | 2,7 | 191 | 42000 | 42,1 | 197,8 | 170°C, 20 bar $H_2$, LHSV 5,0 $h^{-1}$ | Rieselbett |
| 10 | 54% Ni/SiO$_2$ 0,45-1,0 mm | 0,03 | 387 | 420 | 22,3 | 198,3 | 120°C, 140 bar $CO_2$, überstöchiom. $H_2$ LHSV 5,0 $h^{-1}$ | gemäß Erfindung |
| 10 | " | 0,01 | 203 | 223 | 23,5 | 197,2 | 170°C, 20 bar $H_2$ LHSV 5,0 $h^{-1}$ | Rieselbett |
| 7 | 2% Pd/TiO$_2$ | 0,12 | 57,8 | 2890 | 0,16 | 202,6 | 170°C, 20 bar $H_2$, LHSV 1,07 $h^{-1}$ | gemäß DE 41 00 502 |
| 8 | 2% Pd/C | 0,23 | 57,2 | 5720 | 0,74 | 203,4 | dest. Fettsäure | |
| 9 | 0,5% Pd/Al$_2$O$_3$ | 0,10 | 48 | 9600 | 39 | – | 60°C, 21,1 bar $H_2$, LHSV 1 $h^{-1}$ Fettsäure (Ölsäure) | gemäß DOS 23 10 958 |

EP 0 745 116 B1

Tabelle 8  Vergleich verschiedener Katalysatorsysteme in der überkritischen bzw. Rieselbetthärtung

| Kat.-Nr. | Katalysator-bezeichnung/ Korndurch-messer | Hydrier-aktivität A | JZ-Abnahme $[h^{-1}]$ | spezifische JZ-Abnahme $[1/h \cdot gaM]$ | cis/trans-Isomerisie-rung B | Reaktionsparameter | Verfahren |
|---|---|---|---|---|---|---|---|
| 11 | Pd-Monolith | 2,00 | 530 | 6800 | 0,27 | 60°C, 100 bar $CO_2$ überstöchiometrisch $H_2$ LHSV 10 $h^{-1}$ Linolsäurethylester | gemäß Erfindung |
| 12 | " | 1,79 | 472 | 6058 | 0,38 | 170°C, 20 bar $H_2$, LHSV 10 $h^{-1}$ Linolsäureethylester | Rieselbett-bedingungen |

**Patentansprüche**

1. Verfahren zum kontinuierlichen Hydrieren von ungesättigten Fetten, Fettsäuren oder Fettsäureestern an einem geformten Katalysator im Festbett,
   **dadurch gekennzeichnet,**
   daß die Fette, Fettsäuren oder Fettsäureester zusammen mit dem für die Hydrierung benötigten Wasserstoff und in Gegenwart eines überkritischen Mediums oder Lösungsmittels an einem Katalysator umgesetzt werden und daß anschließend die Fette oder Fettsäuren durch Entspannen des überkritischen Mediums bzw. Lösungsmittels von diesem abgetrennt werden.

2. Verfahren nach Anspruch 1,
   **dadurch gekennzeichnet,**
   daß die Hydrierung am Katalysator bei Temperaturen zwischen der kritischen Temperatur $T_{kr}$ des Lösungsmittels und $7 \cdot T_{kr}$, und bei Drucken zwischen dem 0,8-fachen des kritischen Drucks $P_{kr}$ des Lösungsmittels und $6 \cdot P_{kr}$, durchgeführt wird.

3. Verfahren nach Anspruch 2,
   **dadurch gekennzeichnet,**
   daß als Lösungsmittel überkritisches Kohlendioxid, Stickstoffmonoxid, Distickstoffmonoxid, Propan oder Pentan oder binäre oder ternäre Gemische davon, gegebenenfalls unter Zusatz von Modifikatoren, verwendet werden.

4. Verfahren nach Anspruch 3,
   **dadurch gekennzeichnet,**
   daß als Katalysatoren Platingruppenmetalle, Nickel oder Kupfer gegebenenfalls zusammen mit Promotoren auf geformten Trägern eingesetzt werden.

5. Verfahren nach Anspruch 4,
   **dadurch gekennzeichnet**,
   daß die Träger kugelförmig sind und Durchmesser im Bereich zwischen 0,1 und 3,0 mm aufweisen und daß die Platingruppenmetalle auf diesen Trägern in einer äußeren Schale mit einer Dicke von 10 bis 40 μm aufgebracht sind.

6. Verfahren nach Anspruch 5,
   **dadurch gekennzeichnet**,
   daß es sich bei dem Material der Träger um Organosiloxan-Polykondensate, um Organosiloxanamin-Copolykondensate oder um polymere, sekundäre und/oder tertiäre Organosiloxanaminverbindungen handelt.

7. Verfahren nach Anspruch 3,
   **dadurch gekennzeichnet,**
   daß der Katalysator als Beschichtung auf einem inerten monolithischen Tragkörper in Form einer Schaumkeramik oder eines metallischen oder keramischen Wabenkörpers aufgebracht ist.

8. Verfahren nach Anspruch 3,
   **dadurch gekennzeichnet,**
   daß der Katalysator zu einem monolithischen Wabenkörper verformt ist.


**Claims**

1. Process for the continuous hydrogenation of unsaturated fats, fatty acids or fatty acid esters on a shaped catalyst in a fixed bed,
   characterised in that
   the fats, fatty acids or fatty acid esters are reacted on a catalyst together with the hydrogen required for hydrogenation in the presence of a supercritical medium or solvent and that the fats or fatty acids are subsequently separated from the supercritical medium or solvent by depressurisation thereof.

2. Process according to claim 1,
   characterised in that

hydrogenation is performed on the catalyst at temperatures between the critical temperature $T_{cr}$ of the solvent and $7 \cdot T_{cr}$ and at pressures between 0.8 times the critical pressure $P_{cr}$ of the solvent and $6 \cdot P_{cr}$.

3. Process according to claim 2,
characterised in that
supercritical carbon dioxide, nitrogen monoxide, dinitrogen monoxide, propane or pentane or binary or ternary mixtures thereof, optionally with the addition of modifiers, are used as the solvent.

4. Process according to claim 3,
characterised in that
platinum group metals, nickel or copper, optionally together with promoters, on shaped supports are used as the catalysts.

5. Process according to claim 4,
characterised in that
the supports are spherical and have diameters in the range between 0.1 and 3.0 mm and that the platinum group metals are applied onto these supports as an external shell of a thickness of 10 to 40 µm.

6. Process according to claim 5,
characterised in that
the support material comprises organosiloxane polycondensation products, organosiloxaneamine copolycondensation products or polymeric, secondary and/or tertiary organosiloxaneamine compounds.

7. Process according to claim 3,
characterised in that
the catalyst is applied as a coating onto an inert monolithic support in the form of ceramic foam or a metallic or ceramic honeycomb.

8. Process according to claim 3,
characterised in that
the catalyst is shaped into a monolithic honeycomb.

**Revendications**

1. Procédé d'hydrogénation en continu de graisses, d'acides gras ou d'esters d'acides gras insaturés sur un catalyseur moulé en lit fixe,
caractérisé en ce que
les graisses, les acides gras ou les esters d'acides gras avec l'hydrogène nécessaire au durcissement et en présente d'un milieu surcritique ou d'un solvant sont mis à réagir sur un catalyseur et, qu'ensuite on sépare les graisses, acides gras ou esters d'acides gras par détente du milieu ou du solvant surcritique pour éliminer ces derniers.

2. Procédé selon la revendication 1,
caractérisé en ce qu'
on réalise l'hydrogénation sur le catalyseur à des températures comprises entre la température critique ($T_{kr}$) du solvant et ($7.T_{kr}$), et à des pressions comprises entre 0,8 fois la pression critique ($P_{kr}$) du solvant et ($6.P_{kr}$).

3. Procédé selon la revendication 2,
caractérisé en ce qu'
on utilise comme solvant du dioxyde de carbone surcritique, du monoxyde d'azote, de l'hémioxyde d'azote, du propane ou du pentane ou leurs mélanges binaires ou ternaires, le cas échéant en ajoutant des modifiants.

4. Procédé selon la revendication 3,
caractérisé en ce qu'
on utilise, comme catalyseurs, des métaux du groupe du platine, du nickel ou du cuivre, le cas échéant en présence de promoteurs sur des supports moulés.

5. Procédé selon la revendication 4,

caractérisé en ce que

les supports sont de forme sphérique et présentent un diamètre compris entre 0,1 et 3,0 mm et que les métaux du groupe du platine sont déposés sur ces support en une couche extérieure ayant une épaisseur de 10 à 40 $\mu$m.

6. Procédé selon la revendication 5,
caractérisé en ce qu'
en ce qui concerne le matériau des supports, il s'agit de polycondensats d'organosiloxanes, des copolycondensats d'organosiloxanamine ou de composés polymères d'organosiloxanamines secondaires et/ou tertiaires.

7. Procédé selon la revendication 3,
caractérisé en ce que
le composé est disposé comme revêtement sur un support monolithique inerte sous forme d'une céramique expansée ou d'un corps en nid d'abeille métallique ou céramique.

8. Procédé selon la revendication 3,
caractérisé en ce que
le catalyseur est moulé en un corps en nid d'abeille.

Figur 1